# FASCICULE DE BREVET EUROPEEN

(11) **EP 0 975 324 B1**
(45) Date de publication et mention de la délivrance du brevet: **03.04.2002**
(21) Numéro de dépôt: 98941509.6
(22) Date de dépôt: 31.07.1998
(51) Int. Cl.: A61K 7/48

(54) **COMPOSITION COSMETIQUE ANTI-DESQUAMANTE ET PROCEDE DE FABRICATION D'UNE TELLE COMPOSITION**
ABSCHUPPENDE ZUSAMMENSETZUNG UND VERFAHREN ZUR HERSTELLUNG DIESER ZUSAMMENSETZUNG
ANTI-DESQUAMATIVE COSMETIC COMPOSITION AND METHOD FOR MAKING SAID COMPOSITION

(30) Priorité: 01.08.1997 FR 9710107
(43) Date de publication de la demande: 02.02.2000
(73) Titulaire: GATTEFOSSE S.A., 69800 Saint Priest (FR)
(72) Inventeur: CHAUTARD, Alain, F-69500 Bron (FR); BOURLAND, Jacqueline, F-75001 Paris (FR); ROUX, Denis, F-38360 Noyarey (FR)
(74) Mandataire: Vuillermoz, Bruno
(86) Numéro de dépôt international: FR9801708
(87) Numéro de publication internationale: WO9906019

(56) Documents cités:
- EP-A- 0 473 502
- DATABASE WPI Derwent Publications Ltd., London, GB; AN 92253484 XP002063135 SHISEIDO: "Safe external skin agent whitening skin and preventing rough skin-contains at least one protease inhibitor, e.g. soybean trypsin inhibitor and at least one ketose" & JP 04 169514 A (SHISEIDO), 17 juin 1992
- CHEMICAL ABSTRACTS, vol. 119, no. 26, 27 décembre 1993 Columbus, Ohio, US; abstract no. 278371, SAWAKI SHIGERU E.A.: "Skin aging-preventing cosmetics" XP002063134 & JP 05 221844 A

## Description

### Domaine de l'Invention

L'invention se rapporte à une composition cosmétique anti-desquamante. Elle concerne également le procédé de fabrication d'une telle composition.

### Technique antérieure

Les espaces du stratum corneum ou couche cornée, c'est-à-dire la couche superficielle de l'épiderme sont occupés par les cornéodesmosomes et les feuillets lipidiques, qui assurent la cohésion cellulaire et la fonction "barrière cutanée".

La desquamation proprement dite, résulte d'un double mécanisme, à savoir destructuration des membranes lipido-protéiques cornéocytaires et dégradation enzymatique des cornéodesmosomes sous l'action de diverses enzymes (lipases, sphingomyélinases et protéases). Toutefois, le processus de desquamation apparaît prioritairement contrôlé par deux enzymes de la famille des Sérines-Protéases, une enzyme Trypsine-like et une enzyme Chymotrypsine-like.

Dans une peau normale, la desquamation met en jeu l'élimination de cornéocytes individuels à la surface de la peau. C'est un processus parfaitement régulé qui contrebalance la production continuelle de cornéocytes par différentiation des kératinocytes, permettant ainsi de maintenir l'épaisseur de la couche cornée constante.

Le stratum corneum remplit en outre une fonction de « barrière » grâce à son organisation en double compartimentation, qui met en jeu une répartition régulière des cornéocytes ou poches hydrophiles au sein d'un ciment lipidique hydrophobe.

Lorsque la peau est soumise à différents facteurs environnementaux tels que des agents irritants, les tensioactifs, les savons, le sel de mer, le vent, les écarts de températures, un phénomène d'hyper-desquamation réactionnelle se produit, conduisant à une exfoliation excessive qui peut induire des lésions dans le stratum cornéum par perte de cohésion, et altérer ainsi la fonction barrière de celui-ci.

La perte de structure de cet ensemble conduit à une peau qui perd sa capacité à contrôler le flux trans-épidermal et qui de ce fait, devient sèche.

Pour lutter contre ce type de « peau sèche induite », les techniques mises en oeuvre actuellement consistent à réhydrater l'épiderme par un agent hydratant, et/ou à reconstruire le milieu intercellulaire à partir de corps gras, notamment de lipides du type céramide.

Même si ces techniques permettent d'endiguer le phénomène, elles ne permettent en aucun cas de réduire l'apparition des squames. En d'autres termes, les produits utilisés n'agissent pas directement sur la régulation de la desquamation qui entraîne la rugosité des peaux sèches.

Par ailleurs, de telles méthodes font appel à l'utilisation de deux types de produits différents mis en oeuvre conjointement, augmentant ainsi le coût du traitement.

De plus, dans le cadre de la recherche fondamentale, on a mis en évidence que la chymostatine synthétisée chimiquement inhibait certaines sérines protéases, plus spécifiquement la chymotrypsine. Cette molécule d'origine chimique est beaucoup trop toxique pour être appliquée au domaine de la cosmétologie et ne peut donc être utilisée qu'in-vitro, dans le cadre de la recherche fondamentale.

Le problème que se propose de résoudre l'invention est donc de mettre en oeuvre une composition cosmétique anti-desquamante susceptible de réguler la dégradation des cornéodesmosomes et l'excès de desquamation identifié dans certains désordres cutanés, tels que les peaux sèches induites par des facteurs environnementaux.

Cette composition cosmétique pourra avoir également une activité protectrice, préventive afin d'éviter l'assèchement des peaux saines lors d'expositions prolongées aux facteurs responsables d'une stimulation de l'activité protéasique. Il contribuera, dans ce cas, au maintien de l'hydratation naturelle en préservant l'intégrité de la barrière cornée.

Un autre problème que se propose de résoudre l'invention est de fournir un produit qui soit efficace à lui seul sans faire appel à un produit complémentaire.

### Exposé de l'invention :

Pour réguler tout phénomène d'hyperdesquamation de la peau, l'invention propose une composition cosmétique anti-desquamante à base d'anti-sérine protéases, caractérisée en ce que les anti-sérine protéases sont choisies dans le groupe comprenant l'anti-α-chymotrypsine extraite de céréales.

En d'autres termes, l'anti-α-chymotrypsine agit en inhibant la chymotrypsine synthétisée dans le stratum granulosum puis libérée à la jonction stratum corneum - stratum granulosum. Dans le stratum corneum, elle est localisée uniquement dans les espaces inter-cellulaires, associée aux bicouches ceramidiques et sur l'enveloppe cornée des cornéocytes.

Rien ne suggérait à l'homme du métier que des anti-sérine protéases du type anti-α-chymotrypsine d'origine céréalière soient capables d'inhiber des sérine protéases humaines.

Par ailleurs, l'anti-α-chymotrypsine extraite de céréale, outre son action inhibitrice de la chymotrypsine permet de diminuer la réticulation du collagène et donc empêcher celui-ci de perdre ses caractéristiques d'élasticité.

En d'autres termes, l'anti-α-chymotrypsine d'origine céréalière permet non seulement de lutter contre la desquamation, mais également contre le vieillissement de la peau en maintenant la structure cutanée.

Par ailleurs, pour renforcer sont activité anti-desquamante, la composition de l'invention comprend en outre une anti-trypsine.

L'anti-trypsine agit en inhibant la trypsine présente dans le compartiment intra-cellulaire en association avec des filaments de kératine.

Comme source d'extraction céréalière d'anti-sérine protéases, on utilise avantageusement les grains de blé.

Toutefois, et selon un autre caractéristique de l'invention, on utilise comme source d'extraction céréalière d'anti-sérine protéases des céréales stressés pu génétiquement modifiés, soit par sélection naturelle, soit par incorporation artificielle de gène.

On a en effet constaté que de façon tout a fait surprenante, les céréales stressés ou génétiquement modifiés présentaient une concentration élevée en anti-sérine protéases.

Selon un mode préféré de réalisation de l'invention, on met en oeuvre des grains de blé stressé ou des grains de blé génétiquement modifié, soit par sélection naturelle, soit par incorporation artificielle de gène.

Par « blé stressé », on désigne un blé ayant été privé d'eau au cours, de sa croissance et développant ainsi un caractère important de résistance aux maladies et aux accidents, notamment par rapport aux facteurs climatologiques.

Par « sélection naturelle», on désigne un blé obtenu par croisement.

L'invention concerne également le procédé pour la fabrication d'une telle composition cosmétique anti-desquamante qui se caractérise en ce que :
- on broie d'abord des céréales afin d'obtenir une farine ;
- puis on soumet la farine obtenue à une extraction au tampon phosphate, à froid;
- on centrifuge et clarifie ensuite l'extrait obtenu avant de le soumettre à une filtration ;
- on élimine le tampon phosphate ;
- on récupère la solution obtenue.

L'élimination du tampon phosphate est réalisée par les techniques connues de l'homme du métier telles que dialyse ou encore ultrafiltration.

La solution ou filtrat peut ensuite être transformée en différentes formes.

Ainsi, selon une première forme de réalisation, on ajoute dans la solution un agent stabilisant des anti-sérine protéases. Avantageusement, on fabrique une solution glycérinée à 30 % en volume.

Selon une seconde forme de réalisation, la solution est gélifiée, par addition notamment d'un polymère naturel tel qu'une gomme xanthane.

Enfin, la solution obtenue après filtration peut être séchée par lyophilisation, atomisation ou étuve à vide, et ce, afin d'obtenir une poudre.

Les trois formes obtenues, que ce soit solution, gel ou poudre peuvent ensuite être incorporées, de façon connue, au sein d'une composition cosmétique.

Pour obtenir une concentration plus importante en anti-α-chymotrypsine et surtout diminuer la concentration de protéase gênante du type trypsine-like, on fait suivre l'étape d'extraction de la farine de blé par une étape de précipitation par le sulfate d'ammonium.

Avantageusement, on fait suivre l'étape d'extraction par une double étape de précipitation par le sulfate d'ammonium pour éliminer complètement les protéases-trypsine-like.

Lorsqu'on traite un faible volume de farine, on procède après chaque précipitation à une dialyse de la solution afin d'éliminer le sulfate d'ammonium.

Lorsqu'il s'agit de volume plus important, on procède à une ultrafiltration.

L'invention et les avantages qui en découlent ressortiront mieux des exemples de réalisation qui suivent.

### Manière de réaliser l'invention

### Exemple 1

### Préparation d'un extrait de grains de blé

On utilise comme matière première un blé stressé connu sous la dénomination « Charly » .

On broie tout d'abord 150 kg de grains de blé. On procède ensuite à une extraction à froid de la farine obtenue en utilisant un solvant comprenant :
- 10 kg de phosphate disodique
- 0,7 kg de phosphate monosodique
- 1000 litres d'eau osmosée

L'extraction est effectuée pendant au moins trois heures.

On centrifuge ensuite l'extrait obtenu sur une centrifugeuse continue, du type Rousselet ®.

On procède ensuite à une clarification, puis à une filtration. On élimine le tampon phosphate par ultra filtration et on concentre le produit. On effectue une double précipitation en sulfate d'ammonium (à 250 g/l), chacune étant suivie d'une ultra filtration pour éliminer le sulfate, avant de sécher la solution obtenue, par exemple par atomisation.

On obtient ainsi 200 g d'extrait de grains de blé. On incorpore ensuite une partie de cet extrait à différents types de préparations cosmétiques.

### Préparation d'une crème pour peau hyperdesquamante

Les pourcentages sont donnés en poids de la préparation finale.

On prépare les quatre phases suivantes.

| Phase I : | | |
|---|---|---|
| agent émulsifiant | APIFIL® commercialisé par GATTEFOSSE | 8 % |
| agent épaississant | alcool cétylique | 0,25 % |
| agents émollients | octyldodecyl myristate | 9 % |
| | isostearyl isostearate | 8 % |
| agent adoucissant | VEGETOL ® CALENDULA commercialisé par | |
| | GATTEFOSSE | 4 % |
| | acétate de vitamine E | 0,2 % |

| Phase II : | | |
|---|---|---|
| | eau déminéralisée | 56,7 % |
| agent gélifiant | CARBOPOL 934 commercialisé par | |
| | GOODRICH | 0,2 % |

| Phase III : | | |
|---|---|---|
| agent neutralisant | hydroxyde de sodium (à 10 %) | 0,4 % |

| Phase IV : | | |
|---|---|---|
| agent conservateur | GERMABEN II E commercialisé par ISP | 1 % |
| Extrait de grains de blé en solution glycérinée à 30 % | | 12 % |

Après avoir dispersé le CARBOPOL dans l'eau, on laisse reposer. On ajoute ensuite, sous agitation, la phase II chauffée à 75° dans la phase I chauffée à la même température.

On ajoute ensuite au mélange obtenu la solution d'hydroxyde de sodium, puis on refroidit sous agitation.

Aux environs d'une température de 35°C, on ajoute la phase IV.

On obtient ainsi une crème pour peaux sèches anti-desquamante.

### Préparation d'un sérum

| Phase I : | | |
|---|---|---|
| | eau déminéralisée | 60,6 % |
| | glycérine | 5 % |
| | propylène glycol | 5 % |
| agent gélifiant | KELTROL T commercialisé par KELCO | 0,3 % |
| agent chélatant | EDTA | 0,1 % |
| Extrait de blé grain de blé en poudre | | 1 % |

| Phase II : | | |
|---|---|---|
| | eau déminéralisée | 9,7 % |
| agent gélifiant | JAGUAR HP 60 commercialisé par | |
| | RHONE POULENC | 0,3 % |

| Phase III : | | |
|---|---|---|
| agent conservateur : | GERMABEN II commercialisé par ISP | 1 % |

Après avoir dispersé le KELTROL T dans l'eau, on laisse reposer. On prépare ensuite la solution de JAGUAR. Puis, sous agitation, on ajoute la phase II à la phase I puis la phase III.

On obtient un sérum destiné à la protection des peaux exposées aux détergents.

### Fabrication d'un lait hydratant

| Phase I : | | |
|---|---|---|
| agent émulsifiant | TEFOSE ® 2000 (commercialisé par GATTEFOSSE) | 6 % |
| agents épaississants | acide stéarique | 1 % |
| | GELEOL (commercialisé par GATTEFOSSE) | 1% |
| agent émollient | isostearyl isostearate | 12 % |
| | huile de noyaux d'abricot | 4 % |
| Agent lubrifiant | silicone | 4 % |
| Agent conservateur | PHENONIP ® (commercialisé par NIPA) | 0,6 % |

| Phase II : | | |
|---|---|---|
| | eau déminéralisée | 63 % |
| agent gélifiant | NATROSOL 250 HX PHARMA (commercialisé par AQUALON) | 0,2 % |

| Phase III : | |
|---|---|
| eau déminéralisée | 5 % |
| extrait de grains de blé en poudre | 1 % |

| Phase IV : | |
|---|---|
| Parfum | 0,2 % |

Après avoir dispersé le NATROSOL dans la phase II, on laisse reposer. On agite ensuite sous agitation la phase II chauffée à 75°C dans la phase I également chauffée à 75°C.

On maintient le mélange à cette température pendant deux à trois minutes.

On refroidit ensuite sous agitation et on ajoute les autres constituants à une température d'environ 30°C.

On obtient un lait hydratant anti-desquamant.

### Exemple 2

Dans cet exemple, on a dosé par spectrophotométrie et comparé l'activité anti-chymotrypsine et l'activité trypsine d'un extrait de blé stressé du type Charly et d'un extrait de blé non stressé du type Récital, obtenu selon le procédé de l'invention.

### A/Blé Charly

L'activité anti-chymotrypsine référencée IC 50 par la suite correspond à la concentration d'extrait de blé nécessaire pour inhiber 50 % de l'activité d'une chymotrypsine (échantillon fourni par Boehringer) sur substrat Succ-Arg-Pro-Tyr-pNA.

On a regroupé dans le tableau ci-après les activités anti-α-chymotrypsine et trypsine dosées à partie d'un même extrait auquel on fait subir des étapes de traitement supplémentaires.

Les activités ont ainsi été dosées sur l'extrait suivant :
1 - extrait dialysé
2 - extrait ultra-filtré puis dialysé
3 - culot de précipitation obtenu lors de cette même étape de précipitation
4 - culot de précipitation par le sulfate d'ammonium à une concentration de 250 g/l à pH de 7,6;
5 - culot de précipitation par le sulfate d'ammonium à une concentration de 250 g/l à pH de 6,8 ;
6 - culot de précipitation par le sulfate d'ammonium à une concentration de 250 g/l à pH de 5;
7 - culot de précipitation par le sulfate d'ammonium à une concentration de 350 g/l

| **Essai Pilote** | **Activité anti-chymostrypsine IC 50** | **Activité trypsine µ g trip/ml extrait** |
|---|---|---|
| 1 | 0,18 | 48,5 |
| 2 | 0,25 | 34,4 |
| 4 | 0,15 | 38,5 |
| 5 | 0,13 | 8 |
| 6 | 0,13 | 8,15 |
| 7 | 0,13 | 9 |
| 8 | 0,28 | 13,5 |

Comme le montre le tableau, on constate qu'une précipitation simple au sulfate d'ammonium de la farine de blé permet de diminuer largement l'activité trypsine (8) par rapport au même extrait ayant subi uniquement une opération de dialyse.

Enfin, on a effectué sur le même extrait une double précipitation au sulfate d'ammonium à partir de la farine de blé. On obtient une activité anti-chymotrypsine IC 50 de 0,24 et une activité trypsine de 0. On constate donc que la double précipitation permet de supprimer totalement l'activité trypsine de la composition.

### B/Blé récital

Les mêmes essais réalisés sur un blé Récital montre que l'activité anti-chymotrypsine IC 50 est comprise entre 1 et 0,8.

### Exemple 3

On a réalisé dans cet exemple des essais in vitro permettant de caractériser l'activité d'une composition à base de blé stressé sur la desquamation d'explants cutanés humains.

### Conditions :

- la peau utilisée provient d'une plastie abdominale fraîchement prélevée sur une patiente de 54 ans. Deux fragments de peau provenant du même donneur ont été utilisés.
- Produits testés :
   . 100498 DSA : extrait de blé possédant une activité antichymotrypsine (IC50 = 0,28) et absence d'activité TRYPSINE.
   . FDSA = extrait de blé possédant une activité antichymotrypsine (IC50= 0,15) mais également une activité TRYPSINE.
   . inhibiteur de protéase, l'aprotinine, servant de référence pour son effet inhibiteur d'une hyperdesquamation (250 µg/ml).

### Méthode :

- 50 µl des produits à l'essai et du produit de référence sont appliqués sur la surface de l'épiderme des 2 explants - un lot témoin est non traité. Les explants sont maintenus en survie dans un milieu nutritif, la face épidermique restant au contact de l'air.
- après 18 heures de pré-incubation, un des eux explants est irradié par des UVA puis des UVB afin d'induire une hyperdesquamation pour déterminer si les composés testés sont capables d'inhiber cette desquamation induite.
- le deuxième explant ne subit pas d'irradiation.
- Puis les explants sont à nouveau incubés pendant 24 heures avec les solutions fraîches du produit à l'essai et du produit de référence.
- A la fin du traitement, on recueille les cornéocytes libérés par lavage de la surface des explants. Les cornécytes sont dissociés par un tampon approprié et les échantillons sont déposés sur une feuille de nitrocellulose.
- La détection des structures dérivés des cornéocytes se fait par la technique anticorps polyclonal anti « cornéocyte total » revélés par un conjugué anti-immunoglobuline de souris-peroxydase.
- La peroxydase fixée est révélée par chimioluminescence.
- Les données brutes sont traitées par un logiciel adapté et les comparaisons inter-groupes sont réalisées par analyse de variance, à l'aide du test de comparaison multiple de Dunett.

Les résultats sont donnés dans les tableaux suivants :

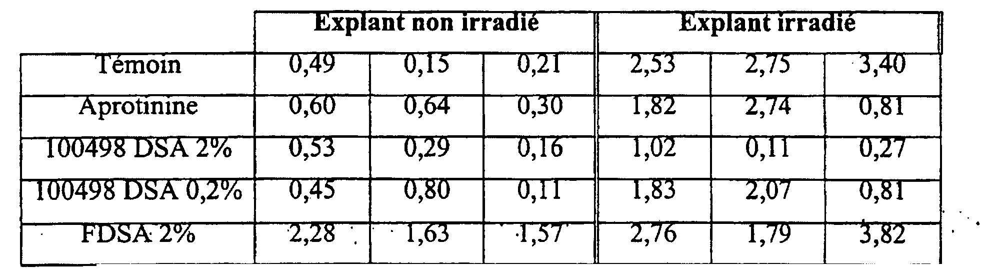

Quantité relative de matériel cornéocytaire détaché en données densitométriques brutes (AU/mm²) désignée D.

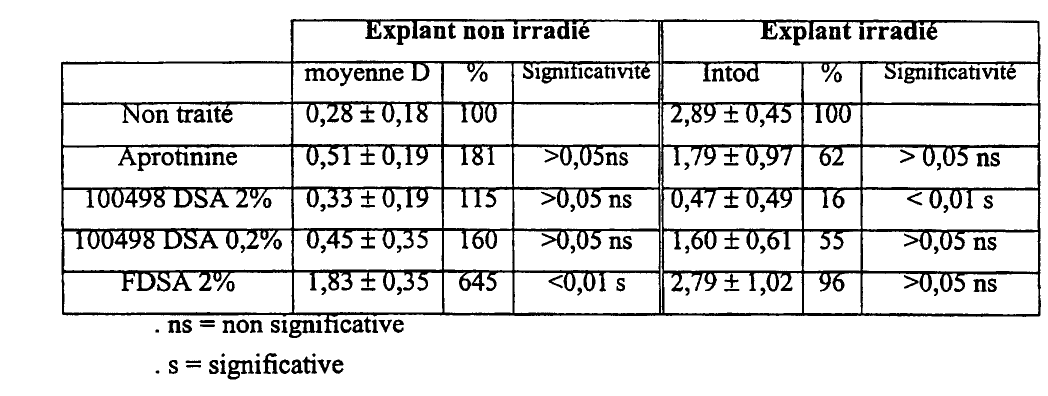

### Conclusion

Le signal obtenu avec les témoins non traités montre que l'irradiation UV a bien induit une hyperdesquamation.

L'inhibiteur de protéase choisi, l'aprotinine entraîne bien une inhibition de la desquamation induite par les UV même si dans les conditions de l'étude, celle-ci n'est pas significative (62 % du témoin irradié).

L'extrait de blé 100498 DSA évalué inhibe également la desquamation induite par les UV, ceci de façon significative à la concentration de 2% (16 % du témoin irradié).

Par ailleurs, il est intéressant de constater que dans des conditions normales de desquamation (explant non irradié), cet extrait de blé évalué n'a pas d'influence sur la desquamation.

Par contre, l'extrait de blé FDSA possédant une activité TRY à un comportement vis à vis de la desquamation totalement différent. Dans des conditions normales, il agit en augmentant la keratolyse et possède donc un effet desquamant lié à la TRYPSINE, alors qu'il ne possède aucun effet sur la desquamation dans des conditions d'hyperdesquamation (pas d'effet antidesquamant).

Ceci confirme donc la necessité d'éliminer l'activité TRYP de l'extrait de blé qui contrecarre l'activité antidesquamante lié à l'antichymotrypsine.

L'invention permet donc d'obtenir des produits sous forme de lait, de crème, de sérum susceptible d'inhiber l'action des sérine protéases intervenant dans la régulation de la desquamation.

En outre, elle permet d'obtenir également un intérêt dans la lutte du vieillissement de la peau.

De plus et surtout, elle procure une anti-serine protéase d'origine naturelle et donc non toxique pour la peau.

## Revendications

1. Composition cosmétique anti-desquamante à base d'anti sérine protéases, **caractérisée en ce que** les anti-sérine protéases sont choisies dans le groupe comprenant l'anti-a-chymotrypsine extraite de céréale.

2. Composition selon la revendication 1, **caractérisée en ce qu'**elle comprend en outre une anti-trypsine.

3. Composition selon les revendications 1 et 2, **caractérisée en ce que** l'anti-α-chymotrypsine et l'anti-trypsine sont extraites de grains de blé.

4. Composition selon la revendication 3, **caractérisée en ce que en ce que** l'anti-α-chymotrypsine et l'anti-trypsine sont extraites de céréales stressés ou génétiquement modifiés.

5. Composition selon la revendication 3, **caractérisée en ce que** les grains sont des grains de blé stressé.

6. Composition selon la revendication 3, **caractérisée en ce que** les grains de blé sont des grains de blé génétiquement modifié, soit par sélection naturelle soit par incorporation artificielle de gène, de sorte à développer un caractère de résistance aux maladies.

7. Procédé pour la fabrication d'une composition cosmétique anti-desquamante selon l'une des revendications 1 à 6, **caractérisé en ce que** :
- on broie d'abord des céréales afin d'obtenir une farine ;
- puis on soumet la farine obtenue à une extraction au tampon phosphate, à froid;
- on centrifuge et clarifie ensuite l'extrait obtenu avant de le soumettre à une filtration ;
- on élimine le tampon phosphate ;
- on récupère enfin la solution obtenue.

8. Procédé selon la revendication 7, **caractérisé en ce que** les céréales sont des grains de blé stressé.

9. Procédé selon l'une des revendications 7 à 8, **caractérisé en ce que** l'extraction à froid est suivie d'au moins une étape de précipitation par le sulfate d'ammonium, puis par une dialyse ou une ultrafiltration.

10. Procédé selon l'une des revendications 7 à 9, **caractérisé en ce qu'**on ajoute à la solution obtenue de la glycérine.

## Patentansprüche

1. Anti-desquamative kosmetische Zusammensetzung auf der Basis von anti-Serin-Proteasen, **dadurch gekennzeichnet, dass** die anti-Serin-Proteasen ausgewählt sind aus der Gruppe, welche aus Getreide extrahiertes anti-α-Chymotrypsin umfasst.

2. Zusammensetzung gemäß Anspruch 1, **dadurch gekennzeichnet, dass** sie außerdem ein anti-Trypsin enthält.

3. Zusammensetzung gemäß den Ansprüchen 1 und 2, **dadurch gekennzeichnet, dass** das anti-α-Chymotrypsin und das anti-Trypsin aus Weizenkörnern extrahiert sind.

4. Zusammensetzung gemäß Anspruch 3, **dadurch gekennzeichnet, dass** das anti-α-Chymotrypsin und das anti-Trypsin aus gestresstem oder genetisch veränderten Getreiden extrahiert wurden.

5. Zusammensetzung gemäß Anspruch 3, **dadurch gekennzeichnet, dass** die Körner gestresste Weizenkörner sind.

6. Zusammensetzung gemäß Anspruch 3, **dadurch gekennzeichnet, dass** die Weizenkörner entweder durch natürliche Auslese oder durch künstlichen Einbau von Genen derart genetisch veränderte Weizenkörner sind, dass sie das Merkmal der Krankheitsresistenz entwickeln.

7. Verfahren zur Herstellung einer anti-desquamativen kosmetischen Zusammensetzung gemäß einem der Ansprüche 1 bis 6, **dadurch gekennzeichnet, dass**:
- man das Getreide zunächst zerkleinert, um ein Mehl zu erhalten;
- man dann das erhaltene Mehl einer Extration mit Phosphatpuffer in der Kälte unterwirft;
- man den erhaltenen Extrakt sofort zentrifugiert und klärt, bevor er einer Filtration unterworfen wird;
- man den Phosphatpuffer entfernt;
- man abschließlich die erhaltene Lösung verwertet.

8. Verfahren gemäß Anspruch 7, dadurch gekennezeichnet, dass die Getreide gestresste Weizenkörner sind.

9. Verfahren gemäß einem der Ansprüche 7 bis 8, dadurch gekenzeichnet, dass der Kälteextraktion mindestens ein Ausfällungsschritt mit Ammoniumsulfat, danach eine Dialyse oder eine Filtration folgt.

10. Verfahren gemäß einem der Ansprüche 7 bis 9, **dadurch gekennzeichnet, dass** man der erhaltenen Lösung Glycerin hinzufügt.

## Claims

1. Anti-desquamating cosmetic composition based on anti-serine proteases, **characterized in that** the anti-serine proteases are chosen from the group comprising anti-α-chymotrypsin extracted from cereal.

2. Composition according to Claim 1, **characterized in that** it also comprises an anti-trypsin.

3. Composition according to Claims 1 and 2, **characterized in that** the anti-α-chymotrypsin and the anti-trypsin are extracted from wheat grains.

4. Composition according to Claim 3, **characterized in that** the anti-α-chymotrypsin and the anti-trypsin are extracted from stressed or genetically modified cereals.

5. Composition according to Claim 3, **characterized in that** the grains are grains of stressed wheat.

6. Composition according to Claim 3, **characterized in that** the wheat grains are grains of wheat which has been genetically modified, either by natural selection or by artificial gene incorporation, so as to develop a property of resistance to diseases.

7. Process for manufacturing an anti-desquamating cosmetic composition according to one of Claims 1 to 6, **characterized in that**:
- cereals are first ground to obtain a flour;
- the flour obtained is then subjected to an extraction with cold phosphate buffer;
- the extract obtained is centrifuged and clarified, after which it is subjected to filtration;
- the phosphate buffer is removed;
- the solution obtained is recovered.

8. Process according to Claim 7, **characterized in that** the cereals are grains of stressed wheat.

9. Process according to either of Claims 7 and 8, **characterized in that** the cold extraction is followed by at least one step of precipitation with ammonium sulphate, and then by a dialysis or an ultrafiltration.

10. Process according to one of Claims 7 to 9, **characterized in that** glycerol is added to the solution obtained.
